# EUROPEAN PATENT SPECIFICATION

(11) **EP 0 772 445 B1**
(45) Date of publication and mention of the grant of the patent: **31.03.2004**
(21) Application number: 95925086.1
(22) Date of filing: 20.07.1995
(51) Int. Cl.: A61K 31/7048, A61K 31/63, A61K 31/635, A61K 31/415, A61K 31/165, A61K 9/02, A61K 9/06, A61K 9/00, A61P 15/02, A61P 31/10, A61P 33/02

(54) **PHARMACEUTICAL COMPOSITIONS, MAINLY VAGINAL SUPPOSITORY, CONTAINING MANY DIFFERENT ACTIVE INGREDIENTS**
PHARMAZEUTISCHE PRÄPARATE, INSBESONDERE OVULA, DIE VIELE VERSCHIEDENE AKTIVE BESTANDTEILE ENTHALTEN
COMPOSITIONS PHARMACEUTIQUES UTILISABLES PRINCIPALEMENT COMME OVULE, RENFERMANT PLUSIEURS INGREDIENTS ACTIFS DIFFERENTS

(30) Priority: 25.07.1994 HU 9402182; 10.10.1994 HU 9402182
(43) Date of publication of application: 14.05.1997
(73) Proprietor: Milankovits, Marton, H-1112 Budapest (HU)
(72) Inventor: Milankovits, Marton, H-1112 Budapest (HU)
(86) International application number: PCT/HU1995/000036
(87) International publication number: WO 1996/003135

(56) References cited:
- EP-A- 0 125 759
- EP-A- 0 241 175
- EP-A- 0 432 638
- GB-A- 742 569
- GB-A- 2 126 086
- US-A- 5 096 889
- HELWIG H., OTTO H.H., Arzneimittel, 8th Edition, Volume 1, Stuttgart, Wissenschaftlichte Verlagsgesellschaft m.b.H.; 1992, pages 6-120 to 6-123; 11-4 to 11-7; 11-9 to 11-13; 29-16 to 29-21.

## Description

This invention relates to novel compositions containing different active ingredients, mainly to a vaginal suppository.

It is well known, that many different vaginal suppositories are commercially available. According to the complaints and clinical picture, to the result of the microbiological examinations, it is the physician who has to select from the compositions available the one to use in the therapy. The situation is the same in case of the ointments. The most frequently used composition types are the following:
1) Most frequently used compositions against vaginal mycosis:
   Canasten (the active ingredient is clotrimazol; bis-phenyl-(2--chlorophenyl)-1-(imidazolyl)-methane; and
   Pimafucin (the active ingredient is natamycin - primaricin).
2) Generally used composition against fungal and protozoa infection:
   Klion D (the active ingredient is metronidazol; 1-(2'-hydroxyethyl)-2-methyl-5-nitroimidazol and myconasol-nitrate).
3) Composition used against protozoa infection:
   Klion vaginal suppository (the active ingredient is metronidazol).
4) Characteristic representative of the composition group which exerts its effects through the disinfective action of iodine:
   Betadine (iodine is released from the carrier).
5) Characteristic representative of the vaginal suppository compositions, which feed the natural flora of the vagina:
   Genia 92 (nutrients, e.g.: folic acid, lactic acid, lactose, lactamine).
   Common disadvantage of the compositions mentioned is that none of them makes possible the complex, that is bactericide (pathogens: aerobe and anaerobe bacteria), and anti-Mobiluncus and anti-Gardnerella, fungicide and anti-protozoa effect simultaneously, and that they have no antiviral effect at all.

This invention relates to the preparation of compositions which make possible the attack of a pathogen from many different directions simultaneously, and which at the same time help the body's antiviral struggle. This way the selection of the most useful composition type, and the necessary very thorough consideration will become simpler, and a really effective composition can be administered to the patient with safety.

The basis of the invention is the discovery, that there is a combination of active ingredients, which makes the above objects possible, and has many other advantages, which will be discussed later in detail.

It was experimentally discovered, that if a composition contains
a) - an antibiotic, preferably chloramphenicol or erythromycin;
b) - sulphonamide, preferably sulphadimidin;
c) - clotrimazol (bis-phenyl-(2-chlorophenyl)-1-imidazolyl-methane) or natamycin or nystatin;
d) - 5-nitro-metronidazol, that is metronidazol or tinidazol or nimorazol, in an appropriate ratio, then this aim is available.

The basis of the discovery according to the invention is that the effect of the antibiotic or sulphonamide ingredient combination is unexpectedly intensified by the other two active ingredients; this effect is true in both directions, for example the antibacterial, especially anti-Chiamydia effect of chloramphenicol and sulphonamide is greatly increasing. Another example for the potentiating effect is the increase of the effect of chloramphenicol and metronidazol against anaerobe pathogens (e.g. B. fragilis); the antibacterial effect of the sulphonamide and metronidazol antibiotics is also unexpectedly potentiated generally against each pathogen bacterium, in the antifungal protection provided by the fourth component. Therefore the spectrum of the effect is "wider" and the effect of the combination is much stronger than it is expected from the single components, and at the same time less amount of substance is enough for the recovery than could be expected from the separate application of the different active ingredients. This means at the same time, that practically there are no harmful side effects to be taken into account using the combination. Another significant advantage of the solution according to the invention is, that the usual resistance does not occur.

In a preferred embodiment according to the invention the composition of the preparation is the following, calculated to one vaginal suppository:
Component A: 0,10-0,15 g;
Component B 0,10-0,15 g;
Component C: 0,090-0,10 g clotrimazol or 0,0225-0,025 g natamycin or 0,1 g nystatin;
Component D: 0,50 - 0,45 g.

The carrier is preferably polyethylene-glycol; the amount of this component supplements the combination of the active ingredients to the necessary amount in case of a 10 unit's package.

As to Component A, that is the antibiotic component, it must be mentioned, that instead of chloramphenicol, oxytetracyclin or erythromycin can be used in the same amount. The latter is especially advantageous, because it has a good effect and its allergic effect is smaller. Its wide spectrum of effect means that it is effective even against Chlamydia trachomatis and can be administered during pregnancy. Other antibiotics can also be used, that can kill Chlamydia trachornatis. The necessary amount of the other antibiotics is the same percentage of the usual general oral dose as in case of the chloramphenicol or oxytetracyclin.

It must be mentioned that instead of sulfadiamidin other sulphonamide can also be used. We refer to sulfadiamidin as to a preferred component, but it can be replaced with an other active ingredient, having a similar structure.

As to the Component C, clotrimazol and nystatin are the most effective among the listed ingredients.

As to the Component D metronidazol is preferred.

The vaginal suppository, having an active ingredient combination according to the invention, is more effective and its effect is qualitatively different from the one that can be reached by the separate administration of the different components (administered in any sequence), and it results in complete recovery even in those cases, when recovery could not be reached by the separate administration of the components. This refers to the following applications of the preparation having the composition according to the invention (the different components have effects potentiating each other's effects):

### Prophylactic

The water of the infected swimming-pool or a new sexual partner cannot cause infection if the suppository is used.

Considering its importance, the preparation is indispensable before any gynaecological operation (especially in case of utero-vaginal interventions) as a prophylactic suppository.

### Treatment of new infection

In case of a new infection, received some hours before the treatment, the use of the preparation results in absolute recovery in 90% of the cases. Since systemic treatment is not needed,
- smaller dose is enough, since it acts directly,
- because of the smaller dose the risk of the possible side effects is minimal, and by stopping the treatment the side effects disappear (in case of the local treatment there was no side effect observed);
- any capacity of medical staff is not needed for treatment of complications;
- you don't have to be afraid of the formation of the resistance of the pathogens against the components-used, because in the local treatment the applied relatively small amount of preparation absolutely kills the pathogens.

### Treatment of chronic vaginitis

For the treatment of the cervicalisation (ectopium) or the slightly positive epithelial differences (for example the slightly acetic acid positive epithelium, or the decrease of the iodine positivity), or slightly pathological epithelium. Presently laser surgery, conventional surgery, cryocoagulation or electro-cauterisation is used. The combined suppository promotes the spontaneous healing of the bleed easily, inflamed portio of the uterus, and as a consequence, the interventions mentioned above will become unnecessary. Constant existence of the inflammation plays a decisive role in the formation of the cancer of the cervix of the uterus. Application of the suppository according to the invention stops the risk of the formation of the cancer of the cervix of the uterus, by stopping the inflammation. One component of the suppository containing the ingredient combination according to the invention, the metronidazole, has anticancer effects, for example as it can be observed during the radiation treatment of the tumours, when it increases the efficacy of the radiation treatment.

In case of P₃ cytological results, after the application of the suppository the laboratory findings improved to P₂, according to our observations, without any exceptions.

### Antiviral effect

The combination preparation according to the invention acts indirectly against viral infections too, possibly by killing all non-viral pathogens and the earlier engaged part of the immunocapacity of the body is liberated against the viruses.

In those cases for example, when because of inflamation and vaginal discharge the treatment of the Condyloma acuminatum - caused by the human papilloma virus - failed, after using suppository containing the active ingredient combination, the local Phodophylin treatment proved to be permanently successful.

Moreover, it was observed that patients having frequently reoccurring herpes genitalis, became permanently free of symptoms following the treatment with the preparation.

The preparation according to the invention can also be administered to pregnant women in the proper dose (in this case mostly erythromycin should be used as antibiotic component).

It is to be noted that the compositions according to the invention were tested in 300-400 cases, carrying out 300-400 control as well. In all case the full microbiological examination was performed including Chlamydia trachomatis and the Mycoplasmas.

Preparation of the composition according to the invention can be carried out by methods well known in the art for the preparation of these kind of compositions, in pharmacies if smaller amounts are to be prepared, or in pharmaceutical factories, if larger amounts are to be prepared.

The following examples illustrate the invention through the composition of some preparations, but do not limit the invention.

### Example 1

| | |
|---|---|
| Chloramphenicol | 0,8 g |
| Sulfadiamidin | 0,12 g |
| Clotrimazol | 0,15 g |
| Metronidazol | 0,40 g |
| Carrier: | polyethylene-glycol |

For one vaginal suppository.

### Example 2

| | |
|---|---|
| Chloramphenicol | 0,1 g |
| Sulfadiamidin | 0,1 g |
| Clotrimazol | 0,1 g |
| Metronidazol | 0,40 g |
| Carrier: | polyethylene-glycol |

For one vaginal suppository.

It gives similar results if some other, generally used substance is used as a carrier, for example butyrum cacao.

Besides the vaginal suppository the active ingredient combination of the present invention can be used for other pharmaceutical preparations, for example for ointments, talc powder, solution, painting solution, or vaginal drops, etc.

The ointment form preparations contain the combination of the active ingredient in the same ratio as in the vaginal suppository, together with ointment base, mainly with yellow vaseline and other components known per se, if required. This preparation is especially effective in case of tissue demages (diabetes mellitus, burning, etc).

The talc powder preparation contains the active ingredient combination in solid form, in given cases with solid carriers, for example with talc, etc.

The solutions, painting solutions and vaginal drops are prepared with the appropriate organic solvent. The solutions, vaginal drops are important mainly in the paediatric gynaecology; these preparations can of course be used for adults in adequate doses.

Any pharmaceutical preparation can contain borax.

The pharmaceutical compositions mentioned above can be prepared by the technologies used in the preparation of the pharmaceutical compositions, with carriers and other ingredients, using the usual amounts.

## Claims

1. Vaginal suppository comprising
a) an antibiotic, preferably chloramphenicol or erythromycin;
b) sulphonamide, preferably sulfadimidin;
c) clotrimazol (bis-phenyl-(2-chlorophenyl)-1-imidazolyl-methane) or natamycin or nystatin; and
d) nitro-metronidazol (nitro-imidazol), that is metronidazol or tinidazol or nimorazol and optionally an adequate carrier;
in the following amounts, calculated for one vaginal suppository:
| | |
|---|---|
| Component a | 0,10-0,15 g; |
| Component b | 0,10-0,15 g; |
| Component c | 0,090-0,10 g clotrimazol or |
| | 0,0225-0,025 g natamycin or |
| | 0,1 g nystatin; |
| Component d | 0,50-0,45 g. |

2. Preparation according to claim 1, further containing borax.

3. Pharmaceutical composition wherein said composition is selected from the group consisting of vaginal suppositories, ointments, solutions, painting solutions, vaginal drops and powders and comprising the following components as active ingredient:
a) an antibiotic, preferably chloramphenicol or erythromycin;
b) sulphonamide, preferably sulfadiamidin;
c) clotrimazol (bis-phenyl-(2-chlorophenyl)-1-imidazolyl-methane) or natamycin or nystatin; and
d) nitro-metronidazol (nitro-imidazol)) that is metronidazol or tinidazol or nimorazol and optionally borax and/or carriers and/or additives.

4. Pharmaceutical preparation comprising
a) a sulphonamide, preferably sulfadiamidin;
b) clotrimazol (bis-phenyl-(2-chlorophenyl)-1-imidazolyl-methane) or natamycin or nystatin; and
c) nitro-metronidazol (nitro-imidazol)) that is metronidazol or tinidazol or nimorazol and optionally borax and/or carriers and/or additives.

## Patentansprüche

1. Vaginales Suppositorium, welches enthält:
a) Antibiotikum, vorzugsweise Chloramphenicol oder Erythromycin;
b) Sulfonamid, vorzugsweise Sulfadimidin;
c) Clotrimazol (Bis-Phenyl-(2-Chlorophenyl)-1-Imidazolyl-Methane) oder Natamycin oder Nystatin; und
d) Nitrometronidazol (Nitroimidazol), das heisst Metronidazol oder Tinidazol oder Nimorazol wahlweise mit einer geeigneten Trägersubstanz, in den folgenden Mengen, gerechnet für ein Suppositorium:
Komponent a) 0,10-0,15 g;
Komponent b) 0,10-0,15 g;
Komponent c) 0,090-0,10 g Clotrimazol oder 0,0225-0,025 g Natamycin oder 0,1 g Nystatin;
Komponent d) 0,50-0,45 g.

2. Zubereitung gemäss Anspruch 1, welche darüber hinaus auch Borax enthält.

3. Weiterhin aus den Gruppen von vaginalen Suppositorien, Salben, Lösungen, Tupfern, valigale Tropfen, Pudern gewählte pharmazeutische Zubereitung, welche die folgenden Wirkstoffkomponenten enthält:
a) Antibiotikum, vorzugsweise Chloramphenicol oder Erythromycin,
b) Sulfonamid, vorzugsweise Sulfadimidin;
c) Clotrimazol (Bis-Phenyl-(2-Chlorophenyl)-1-Imidazolyl-Methane) oder Natamycin oder Nystatin; und
d) Nitrometronidazol (Nitroimidazol), das heisst Metronidazol oder Tinidazol oder Nimorazol wahlweise mit Borax und / oder Trägersubstanzen und / oder Zusatzstoffe.

4. Pharmazeutische Zubereitung, welche enthält:
a) Sulfonamid, vorzugsweise Sulfadimidin;
b) Clotrimazol (Bis-Phenyl-(2-Chlorophenyl)-1-Imidazolyl-Methane) oder Natamycin oder Nytatin; und
c) Nitro-Metronidazol (Nitroimidazol), das heisst Metronidazol oder Tinidazol oder Nimorazol und wahlweise Borax und / oder Trägersubstanzen und / oder Zusatzstoffe.

## Revendications

1. Ovule composé
a) d'un antibiotique, de préférence chloramphénicol ou érythromycine ;
b) de sulfonamide, de préférence sulfadimidine ;
c) clotrimazole (bis-phényl-(2-chlorophényl)-1-imidazolyl-méthane) ou natamycine ou nystatine ; et
d) nitro-métronidazole (nitro-imidazole), soit métronidazole ou tinidazole ou nimorazole et en option un excipient adéquat ;
avec les quantités suivantes pour un ovule
| | |
|---|---|
| composant a | 0,10-0,15 g; |
| composant b | 0,10-0,15 g; |
| composant c | 0,090-0,10 g clotrimazole ou |
| | 0,0225-0,025 g natamycine ou |
| | 0,1 g nystatine |
| composant d | 0,50-0,45 g |

2. Préparation suivant la revendication 1, contenant aussi du borate

3. Composition pharmaceutique, comme indiquée, est sélectionnée à partir d'un groupe qui comprend des ovules, des onguents, des lotions, des badigeons, des gouttes vaginales et des poudres et qui contient les substances actives suivantes :
a) un antibiotique, de préférence chloramphénicole ou érythromycine ;
b) du sulfonamide, de préférence sulfadimidine ;
c) clotrimazole (bis-phényl-(2-chlorophényl)-1-imidazolyl-méthane) ou natamycine ou nystatine ; et
d) nitro-métronidazole (nitro-imidazole), soit métronidazole ou tinidazole ou nimorazole et en option du borate et/ou des excipients et/ou additifs.

4. Préparation pharmaceutique composée
a) d'un sulfonamide de préférence sulfadimidine ;
b) clotrimazole (bis-phényl-(2-chlorophényl)-1-imidazolyl-méthane) ou natamycine ou nystatine ; et
c) nitro-métronidazole (nitro-imidazole), soit métronidazole ou tinidazole ou nimorazole et en option du borate et/ou des excipients et/ou additifs.
